(19) Europäisches Patentamt
European Patent Office
Office européen des brevets

(11) Veröffentlichungsnummer : **0 026 318**
**B1**

(12) # EUROPÄISCHE PATENTSCHRIFT

(45) Veröffentlichungstag der Patentschrift : **26.01.83**

(51) Int. Cl.$^3$ : **C 07 C 39/16**, C 07 C 37/20

(21) Anmeldenummer : **80104914.9**

(22) Anmeldetag : **19.08.80**

(54) **Verfahren zur Herstellung reiner 4,4'-Dihydroxydiphenylalkane bzw. -cycloalkane.**

(30) Priorität : **31.08.79 DE 2935316**

(43) Veröffentlichungstag der Anmeldung : **08.04.81 Patentblatt 81/14**

(45) Bekanntmachung des Hinweises auf die Patenterteilung : **26.01.83 Patentblatt 83/04**

(84) Benannte Vertragsstaaten : **DE FR GB IT NL**

(56) Entgegenhaltungen :
**DE A 2 811 182**
**US A 2 923 744**
**US A 2 932 671**
**US A 4 073 815**

(73) Patentinhaber : **BAYER AG**
**Zentralbereich Patente, Marken und Lizenzen**
**D-5090 Leverkusen 1, Bayerwerk (DE)**

(72) Erfinder : **Meyer, Karl-Heinrich, Dr.**
**Deswatinesstrasse 89**
**D-4150 Krefeld-1 (DE)**
Erfinder : **Bottenbruch, Ludwig, Dr.**
**Woehlerstrasse 5**
**D-4150 Krefeld 1 (DE)**
Erfinder : **Jakob, Wolfgang**
**Am Domacker 81**
**D-4130 Moers (DE)**

**EP 0 026 318 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung reiner 4,4'-Dihydroxydiphenylalkane bzw. -cycloalkane

Die Erfindung betrifft ein besonders einfaches Verfahren zur Herstellung von 4,4'-Dihydroxydiphenylalkanen bzw. -cycloalkanen (Bisphenolen) in solcher Reinheit, daß sie direkt zur Erzeugung hochwertiger Kunststoffe eingesetzt werden können.

Bisher hat nur die Herstellung von Bisphenol A (2,2-Bis(4-hydroxyphenyl)-propan) durch Umsetzung von Aceton mit überschüssigem Phenol in Gegenwart saurer Kondensationsmittel technische Bedeutung. Die Reaktion kann mit Mercaptoverbindungen katalysiert werden. Als saure Kondensationsmittel verwendete man zuerst starke Säuren wie Schwefel- oder Salzsäuren, die aber wegen ihrer stark korrodierenden Wirkung und thermischen Schädigung des Produkts restlos aus dem Reaktionsgut entfernt werden müssen. Heute verwendet man meist wasserfreie unlösliche Sulfonsäuregruppen enthaltende Kationenaustauscherharze als saure Kondensationsmittel, weil sie sich von dem flüssigen Reaktionsgemisch vollständig abtrennen lassen. Zur Umsetzung wird eine flüssige Mischung aus Aceton und Phenol durch den festen Ionenaustauscher geleitet und das darin gelöste gebildete Bisphenol isoliert (vgl. DE-AS 1 186 834).

Bei allen diesen Herstellungsverfahren für Bisphenol A entstehen unerwünschte Nebenprodukte, insbesondere o,p-Bisphenol, andere Kondensate wie Trisphenol, Chromane, Indane und höher kondensierte dunkel gefärbte Harze. Nach 6 Stunden bei 70 °C bildet sich im Austauscherbett beispielsweise Reaktionsprodukte etwa folgender Zusammensetzung :

| | |
|---|---|
| Bisphenol A (p,p-Verbindung) | 76,2 % |
| Nebenprodukte : | |
| o,p-Bisphenol | 12,8 % |
| Indan- und Spiroindan-Verbindungen | 4,1 % |
| Chromane | 1,6 % |
| Trisphenol | 1,3 % |
| sonstige | 4,0 % |

Die Nebenprodukte, besonders mono- und trifunktionelle sowie verfärbende müssen vor der Umwandlung in Kunststoffe, z.B. Polycarbonate, entfernt werden, zum Beispiel durch Kristallisation von Bisphenol-Phenol-Addukten aus phenolischer Lösung. Die Nebenprodukte bleiben dabei in der Mutterlauge und können entweder in alkalisch-wäßriger Lösung thermisch zu Phenol und Aceton zurückgespalten oder zur teilweisen Umlagerung zum p,p-Bisphenol in den Reaktionsprozeß zurückgeführt werden.

Andere Reinigungsverfahren, beispielsweise das der US-PS 2 923 744, beruhen auf Umkristallisieren oder Extrahieren mit verschiedenen Lösungsmitteln oder Hochvakuumdestillation, erfordern aber sämtlich großen Aufwand.

Es entstehen weniger Nebenprodukte, wenn man das anfangs flüssige Reaktionsgemisch im Ionenaustauscherbett möglichst weitgehend auskristallisieren läßt. Es ist dann aber schwierig, Kristallisat und Austauscherharz ohne Schädigung voneinander zu trennen.

Gegenstand der Erfindung ist ein Verfahren zur Herstellung von Bisphenolen durch Kondensation von aliphatischen oder cycloaliphatischen Ketonen mit Phenolen mit freier p-Stellung in Gegenwart von aromatischen Sulfonsäuren als Kondensationsmittel und einem Lösungsmittel, das gekennzeichnet ist durch die folgenden Maßnahmen :

1) Verwendung eines organischen Lösungsmittels nämlich eines aliphatischen, cycloaliphatischen oder aromatischen Kohlenwasserstoffes oder entsprechenden Halogenkohlenwasserstoffes oder des Phenols, das zur Herstellung der Bisphenole eingesetzt wird, in dem die aromatischen Sulfonsäuren gut und das gebildete Bisphenol bzw. sein Phenoladdukt möglichst wenig löslich sind.

2) Verwendung von 0,3 bis 3 Mol pro Mol Keton einer aromatischen Sulfonsäure der Formeln III und IV

(III) 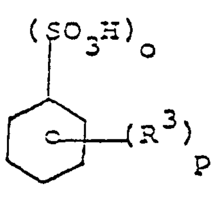 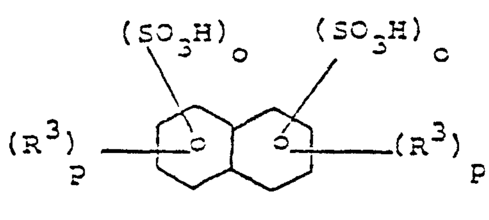 (IV)

in denen

$R^3$ ein inerter organischer Rest ist und
o für eine ganze Zahl von 1 bis 4 und
p für eine ganze Zahl von 0 bis 4 steht.

3) Umsetzen bei einer Temperatur, bei der das entstandene Bisphenol als solches oder als Phenol-Addukt während oder am Ende der Reaktion möglichst weitgehend auskristallisiert.

4) Isolieren des kristallin abgeschiedenen Bisphenols bzw. seines Addukts und

5) Entfernen von anhaftendem Phenol oder Lösungsmittel.

Das so gewonnene Bisphenol ist von überraschend hoher Reinheit und kann direkt zur Herstellung von Polycarbonat- oder Triazinharzen eingesetzt werden.

Die erfindungsgemäß verwendeten Sulfonsäuren binden zwar erhebliche Wassermengen aber ihre Wirksamkeit als Kondensationsmittel nimmt mit steigendem Wassergehalt ab. Man muß daher die Sulfonsäuren weitgehend entwässern oder größere Mengen einsetzen. Besonders günstig ist es, das Reaktionswasser — ein Mol pro Mol Bisphenol — laufend abzudestillieren. Dies ist besonders empfehlenswert, wenn das Verfahren kontinuierlich durchgeführt wird. Um das Wasser abzudestillieren kann man übliche Methoden der Vakuumdestillation und der Azeotropdestillation von Lösungsmittel-Wassergemischen benutzen.

In der kontinuierlichen Ausführungsform des Verfahrens wird das flüssige Reaktionsgemisch aus Lösungsmittel, Sulfonsäure, überschüssigem Phenol und kleineren Mengen gelöster Reaktionsprodukte im Kreis geführt, welchem fortlaufend Phenol und Keton zugeführt und aus welchem reines kristallines Reaktionsprodukt sowie das Reaktionswasser entnommen werden.

Das erfindungsgemäße Verfahren ist bevorzugt für die Herstellung von Bisphenol A geeignet, es können aber auch andere sehr verschiedene Bisphenole hergestellt werden. Als Ausgangsverbindungen lassen sich aliphatische und cycloaliphatische Ketone der Formel (I)

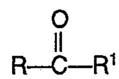

(I)

in der

R und $R^1$ gleich oder verschieden, für einen $C_1$-$C_{10}$-Alkylrest oder einen $C_6$-$C_8$-Cycloalkylrest stehen, bevorzugt einsetzen. Besonders geeignet sind Ketone wie Aceton, Methylethylketon, Methyl-n-propylketon und Cyclohexanon.

Als Phenole mit einer freien p-Stellung werden vorzugsweise Verbindungen der allgemeinen Formel (II) verwendet :

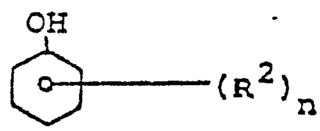

(II)

in der

$R^2$ für einen $C_1$-$C_8$-Alkylrest, vorzugsweise Methyl-, tert.-Butyl-, Isopropyl-, und

n für 0 oder eine ganze Zahl von 1 bis 4 stehen.

Bei diesem Verfahren werden die Phenole stets im Überschuß eingesetzt, vorzugsweise jedoch zwischen 3 und 15 Molen Phenol pro Mol Keton.

Als saure Kondensationsmittel eignen sich besonders alle wasserbindenden aromatischen Mono- und Polysulfonsäuren der allgemeinen Formeln (III) und (IV)

(III) 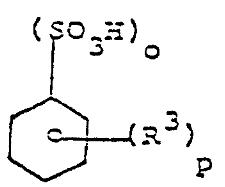 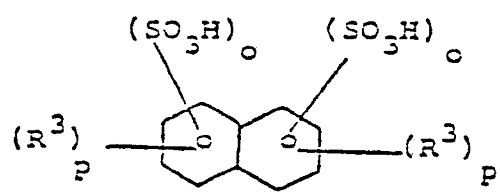 (IV)

in denen

o für eine ganze Zahl von 1 bis 4 und

p für eine ganze Zahl von 0 bis 4 steht und

$R^3$ ein aliphatischer Rest mit $C_1$-$C_{12}$, ein cycloaliphatischer oder aliphatisch-aromatischer Rest, oder Cl, Br, OH, SH, $OR^4$, $SR^4$, $SO_2R^4$, $NO_2$, COOH und $R^4$ ein aliphatischer oder aromatischer Kohlenwasserstoffrest, der inerte Substituenten tragen kann, ist Besonders bevorzugt ist Phenolsulfonsäure.

Diese Sulfonsäuren werden als Kondensationsmittel für die Bisphenolherstellung in Mengen zwischen 0,3 und 3 Molen pro Mol Keton eingesetzt.

Die Reaktionsgeschwindigkeit kann durch Zusatz der bekannten schwefelhaltigen Katalysatoren beschleunigt werden. Darüber hinaus ist sie abhängig von Art und Menge der Sulfonsäure sowie von der Reaktionstemperatur. Besonders aber wirkt sich, wie bereits erwähnt, der Wassergehalt des Kondensationsmittels auf die Umsetzungsgeschwindigkeit aus. Es ist daher zweckmäßig, die Sulfonsäure von ihrem Einsatz intensiv zu trocknen, z.B. 3 Stunden im Vakuum von 20 mbar bei 80 bis 100 °C.

Das für das Verfahren gewählte Lösungsmittel oder Lösungsmittelgemisch hat die Aufgabe, die als Kondensationsmittel verwendete Sulfonsäure vollständig in Lösung zu halten und gleichzeitig die Ausscheidung des gebildeten Bisphenols bzw. seines Phenol-Adduktes in möglichst reiner, gut filtrierbarer Form zu bewirken. Hierzu eignen sich viele aliphatische, cycloaliphatische und aromatische Kohlenwasserstoffe und Halogenkohlenwasserstoffe wie Ligroin, Cyclohexan, Benzol, Toluol, Xylol, Chlorbenzol, o-Dichlorbenzol, Methylenchlorid, 1,2-Dichlorethan, soweit sie Bisphenole und/oder ihre Addukte schlecht, Sulfonsäuren jedoch gut lösen. Besonders eignen sich hierzu die Phenole, die zur Herstellung von Bisphenolen eingesetzt werden, wenn man sie in genügendem Überschuß verwendet.

Beispiel 1

Zur Untersuchung, welchen Einfluß die Menge des sauren Kondensationsmittels auf Ausbeute und Reinheit des gebildeten Bisphenol hat, wurden in Parallelansätzen unterschiedliche Mengen frisch destillierter Benzolsulfonsäure ($Kp_{0,2}$ 142 bis 144 °C) jeweils in einem Gemisch von 180 g Phenol, 30 g Aceton, 100 g Toluol und 0,4 g β-Mercaptopropionsäure in einem 500 ccm-Rührkolben bei 45 °C homogen gelöst und 6 Stunden lang unter Rühren bei dieser Temperatur gehalten.

Der entstandene Kristallbrei wurde anschließend auf einer Nutsche abfiltriert und die Kristalle mit der gleichen Gewichtsmenge Toluol gewaschen, indem man die Waschflüssigkeit in mehreren Portionen verwendete und dabei mit derjenigen Menge Phenol versetzte, die durch die abgetrennten Kristalle in umgesetzter und in anhaftender Form dem Reaktionsgemisch entzogen wurde. Die farblosen Kristalle wurden dadurch völlig von anhaftender Säure befreit. Das in den Kristallen befindliche Reaktionsprodukt und sein Gehalt an p,p-Bisphenol A (p,p-BPA) wurde durch gaschromatographische Analyse ermittelt.

Der in der Mutterlauge gelöste Anteil des Reaktionsproduktes wurde ebenfalls gaschromatographisch untersucht, nachdem er aus der organischen Lösung durch Neutralisierung und Auswaschen der Sulfonsäure mit Wasser und anschließendem Abdestillieren vom Lösungsmittel sowie von nicht umgesetztem Phenol und Aceton als Rückstand erhalten wurde.

Die in der folgenden Tabelle zusammengefaßten Ergebnisse zeigen, daß bei Verwendung größerer Mengen Sulfonsäure die Kristallausbeute bei gleichbleibender hervorragender Bisphenol-A-Qualität stark zunimmt, während sich der in der flüssigen Reaktionsphase gelöst bleibende Bisphenolanteil in Art und Menge praktisch nicht verändert.

Tabelle 1

| Ansatz Nr. | 1 | 2 | 3 | 4 |
|---|---|---|---|---|
| verwendete Benzoesulfonsäure | 1 g | 5 g | 10 g | 20 g |
| Zeit bis Kristallisationsbeginn | 230 Min. | 165 Min. | 100 Min. | 40 Min. |
| Menge Kristallisat nach der Wäsche | 16,4 g | 52,2 g | 91,9 g | 116,2 g |
| Menge des Kond.-produktes i.d. Kristallen | 7,77 g | 24,71 g | 43,26 g | 59,56 g |
| darin Gehalt an p,p-BPA | 99,93 % | 99,92 % | 99,97 % | 99,95 % |
| Mutterlauge-rückstand | 16,32 g | 14,69 g | 17,61 g | 15,74 g |
| darin Gehalt an p,p-BPA | 92,43 % | 91,36 % | 94,76 % | 93,88 % |
| Umsatz bezogen auf eingesetztes Aceton | 20,43 % | 33,42 % | 52,64 % | 63,89 % |

Beispiel 2

In der folgenden Versuchsserie wurden verschiedene Sulfonsäuren in Kombination mit unterschiedlichen Lösungsmitteln verwendet.

a) Eine Lösung von 200 g Phenol, 100 g Leichtbenzin (Kp 90 bis 100 °C), 10 g Dodecylbenzolsulfonsäure (Handelsbezeichnung Marlon AS 3) und 0,2 g β-Mercaptopropionsäure wurde durch Anlegen eines Unterdruckes bei 45 °C in einer Rührapparatur mit Wasserabscheider im Destillationsrücklauf zum Sieden gebracht. Dabei wurden 5 Stunden lang 20 g Aceton eingetropft und das entstehende Reaktionswasser aus dem Destillat abgeschieden. Anschließend wurden die gebildeten Kristalle abfiltriert und mit Leichtbenzin und Phenol (wie bei Beispiel 1) gewaschen.

Danach wurde die Mutterlauge erneut bei 45 °C wie oben 5 Stunden lang mit 20 g Aceton umgesetzt. Nach Abtrennen der neu entstandenen Kristalle wurde die Mutterlauge nochmals mit 20 g Aceton umgesetzt und die Reaktionsmischung erneut getrennt. Die GC-Analyse der aufgearbeiteten Produkte ergab folgendes Bild :

| | |
|---|---|
| 1. Kristallisat BPA-Gehalt | 27,82 g darin 99,91 % p,p-BPA |
| 2. Kristallisat BPA-Gehalt | 37,64 g darin 99,88 % p,p-BPA |
| 3. Kristallisat BPA-Gehalt | 37,86 g darin 99,89 % p,p-BPA |
| Rückstand der letzten Mutterlauge | 17,63 g darin 90,12 % p,p-BPA |

b) Eine Lösung von 282 g Phenol, 22,9 g Aceton, 65 g 1,2-Dichlorethan, 65 g p-Toluolsulfonsäure (Wassergehalt 10 %) und 0,8 g β-Mercaptopropionsäure wurde 3 Stunden bei 45 °C gerührt. Anschließend wurden die entstandenen Kristalle abgesaugt und mit Lösungsmittel und Phenol (wie Beispiel 1) säurefrei gewaschen. Die Mutterlauge wurde anschließend durch Rückflußdestillation im Vakuum mit Wasserabscheidung von Reaktionswasser befreit und erneut mit 22,9 g Aceton umgesetzt. Das Ganze wurde nochmals wiederholt und anschließend die Kristalle und der Rückstand der letzten Mutterlauge analysiert.

| | |
|---|---|
| 1. Kristallisat BPA-Gehalt | 43,57 g darin 99,87 % p,p-BPA |
| 2. Kristallisat BPA-Gehalt | 87,13 g darin 99,42 % p,p-BPA |
| 3. Kristallisat BPA-Gehalt | 88,80 g darin 99,89 % p,p-BPA |
| Mutterlaugenrückstand | 45,53 g darin 92,01 % p,p-BPA |

Beispiel 3

In ähnlicher Weise wie bei den vorherigen Beispielen wurden folgende andere Bisphenole synthetisiert und dabei der Umsatz sowie die Reinheit des kristallinen Reaktionsproduktes ermittelt.

a) 188 g Phenol, 49,1 g Cyclohexan, 10 g Benzolsulfonsäure, 100 g Toluol, 0,3 g β-Mercaptopropionsäure, Reaktionszeit 24 Stunden, Umsatz 79 %, p,p-Bisphenol-Gehalt im kristallinen Reaktionsprodukt : 99,65 % 4,4'-Dihydroxydiphenylcyclohexan-1,1.

b) 188 g Phenol, 36 g Methylethylketon, 20 g Naphthol-1-sulfosäure-4 ($H_2O$-Gehalt 1 %), 0,3 g β-Mercaptopropionsäure, kein zusätzliches Lösungsmittel, Reaktionszeit 24 Stunden, Umsatz 73,5 % ; p,p-Bisphenol-Gehalt des kristallinen Reaktionsproduktes 99,12 %.

c) 235 g 2,6-Dimethylphenol, 30 g Aceton, 36 g p-Phenolsulfonsäure (3 h bei 80 °C und 20 mbar getrocknet), 0,3 g β-Mercaptopropionsäure, kein zusätzliches Lösungsmittel, Reaktionszeit 2 Stunden, Umsatz 51,3 %, p,p-Bisphenol-Gehalt des kristallinen Reaktionsproduktes 99,31 %, 4,4'-Dihydroxy-2,6-Dimethyldiphenylpropan-2,2.

Beispiel 4

Für eine Versuchsserie mit 30-maligem Wiedereinsatz der Mutterlauge wurde eine Lösung von 310 g Phenol, 26 g Aceton, 49 g p-Phenolsulfonsäure (frisch getrocknet) und 0,4 g β-Mercaptopropionsäure 3 Stunden lang bei 45 °C gerührt, dann die Kristalle abgetrennt und mit der entsprechenden Menge Phenol (wie in Beispiel 1) in mehreren Portionen säurefrei gewaschen. Mutterlauge und Waschflüssigkeit wurden danach 1 Stunde bei 7 mbar und 50 °C zur Entfernung des Reaktionswassers abgetoppt und danach erneut mit 26 g Aceton wie oben umgesetzt. Diese Operation wurde bis zum 30. Ansatz wiederholt und anschließend die Kristalle und der Rückstand der letzten Mutterlauge analysiert.

Alle 30 Kristallproben lagen im p,p'-BPA-Gehalt über 99,95 %, z.T. bei 100 %, während der letzte Mutterlaugenrückstand immer noch 90,01 % p,p'-BPA enthielt. Der durchschnittliche Umsatz pro Durchgang lag bei 52 % bezogen auf eingesetztes Aceton. Bezogen auf das gesamte Reaktionsprodukt einschließlich Mutterlaugenrückstand betrug der Anteil an p,p'-BPA 99,49 %. Eine aus den erhaltenen kristallinen Addukten durch Abdestillieren des Phenols hergestellte größere Menge Bisphenol A wurde in folgender Weise auf seine Eignung zur Herstellung von Polycarbonat untersucht : 91 g des Bisphenols wurden in 500 g einer 6,5 %igen wäßrigen Natronlauge gelöst und zusammen mit 900 g Methylenchlorid und 2 g p-tert.-Butylphenol bei 25 °C gerührt. Dazu wurden in 30 Minuten 51,4 g gasförmiges Phosgen eingeleitet und der pH-Wert der Mischung durch vorsichtiges Nachdosieren von wenig 6,5 %iger Natronlauge zwischen 13,0 und 13,5 gehalten. Dann wurden 8 ccm einer 4 %igen wäßrigen Triethylaminlösung zugegeben und weitere 45 Minuten gerührt. Anschließend wurde die untere organische Phase abgetrennt und mehrmals mit destilliertem Wasser gewaschen. Danach wurde das entstandene Polycarbonat in bekannter Weise durch Abdestillieren des Lösungsmittels isoliert, wobei ein sehr helles und klares Produkt mit der relativen Viskosität (0,5 % in Methylenchlorid) von 1,30 erhalten wurde, was für ein hochmolekulares Polycarbonat guter Qualität kennzeichnend ist.

**Ansprüche**

1. Verfahren zur Herstellung von Bisphenolen durch Kondensation von aliphatischen oder cycloaliphatischen Ketonen und Phenolen mit freier p-Stellung in Gegenwart von aromatischen Sulfonsäuren als Kondensationsmittel und einem Lösungsmittel, gekennzeichnet durch die folgenden Maßnahmen :

1) Verwendung eines organischen Lösungsmittels, nämlich eines aliphatischen, cycloaliphatischen oder aromatischen Kohlenwasserstoffes oder entsprechenden Halogenkohlenwasserstoffes oder des Phenols, das zur Herstellung der Bisphenole eingesetzt wird, in dem die aromatischen Sulfonsäuren gut und das gebildete Bisphenol- bzw. Phenol-Addukt möglichst wenig löslich sind.

2) Verwendung von 0,3 bis 3 Mol pro Mol Keton einer aromatischen Sulfonsäure der Formel (III) oder (IV)

(III) 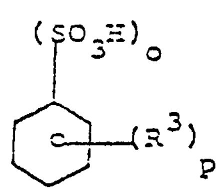 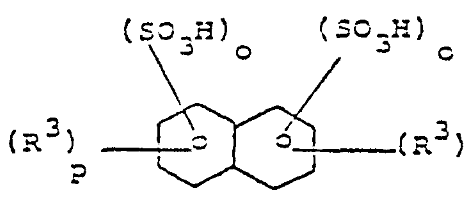 (IV)

in denen

$R^3$ ein inerter organischer Rest ist,

o für eine ganze Zahl von 1 bis 4 und

p für eine ganze Zahl von 0 bis 4 steht.

3) Umsetzen bei einer Temperatur, bei der das entstandene Bisphenol als solches oder als Phenol-Addukt während oder am ende der Reaktion möglichst weitgehend auskristallisiert.

4) Isolieren des kristallin abgeschiedenen Bisphenols bzw. seines Addukts.

5) Entfernen von anhaftendem Phenol oder Lösungsmittel.

2. Kontinuierliches Verfahren nach Anspruch 1, bei dem das Reaktionsgut im Kreis geführt, Bisphenol und Wasser kontinuierlich entnommen und Phenol und Keton kontinuierlich zugefügt werden.

3. Verfahren nach Anspruch 1, in dem das Phenol selbst als Lösungsmittel dient.

## Claims

1. Process for the preparation of bisphenols by condensing aliphatic or cycloaliphatic ketones and phenols having a free p-position in the presence of aromatic sulphonic acids as condensing agent and a solvent, characterised by the following steps :

1) Use of an organic solvent, namely an aliphatic, cycloaliphatic or aromatic hydrocarbon or corresponding halogen-hydrocarbon or the phenol which is used for the preparation of the bisphenols, in which the aromatic sulphonic acids are readily soluble and the bisphenol adduct or phenol adduct formed are sparingly soluble.

2) Use of 0.3 to 3 mols, per mol of ketone, of an aromatic sulphonic acid of the formula (III) or (IV)

(III) 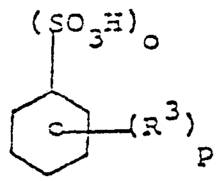 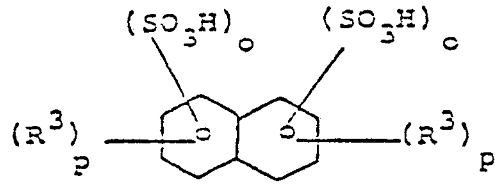 (IV)

in which

$R^3$ is an inert organic radical,

o represents an integer from 1 to 4 and

p represents an integer from 0 to 4.

3) Reaction at a temperature at which the bisphenol formed crystallises as extensively as possible as such or as a phenol adduct during or at the end of the reaction.

4) Isolation of the crystalline separated bisphenol or its adduct.

5) Removal of the adhering phenol or solvent.

2. Continuous process according to Claim 1, in which the reaction mixture is circulated, bisphenol and water being continuously removed and phenol and ketone being continuously added.

3. Process according to Claim 1, in which the phenol itself is used as a solvent.

## Revendications

1. Procédé de production de bisphénols par condensation de cétones aliphatiques ou cycloaliphatiques et de phénols à position para libre en présence d'acides sulfoniques aromatiques utilisés comme agents de condensation et d'un solvant, caractérisé par les mesures suivantes :

1) Utilisation d'un solvant organique, à savoir d'un hydrocarbure aliphatique, cycloaliphatique ou aromatique ou d'un hydrocarbure halogéné correspondant ou du phénol qui est utilisé pour la production des bisphénols, dans lequel les acides sulfoniques aromatiques se dissolvent bien et le produit d'addition de bisphénol ou de phénol formé est aussi peu soluble que possible.

2) Utilisation de 0,3 à 3 moles, par mole de cétone, d'un acide sulfonique aromatique de formule (III) ou (IV) :

(III) 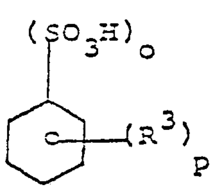 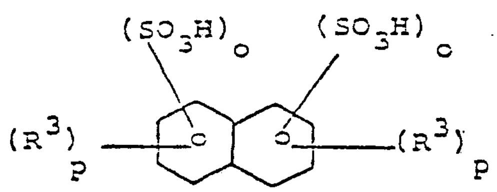 (IV)

où

$R^3$ est un reste organique inerte,

o est un nombre entier de 1 à 4 et

p est un nombre entier de 0 à 4.

3) Réaction à une température à laquelle le bisphénol formé, tel quel ou sous la forme de produit d'addition de phénol, cristallise aussi complètement que possible au cours ou à la fin de la réaction.

4) Isolement du bisphénol ou de son produit d'addition précipité à l'état cristallin.

5) Elimination du phénol ou du solvant adhérent.

2. Procédé continu suivant la revendication 1, dans lequel la matière en réaction est recyclée, du bisphénol et de l'eau sont prélevés en continu et du phénol et de la cétone sont ajoutés en continu.

3. Procédé suivant la revendication 1, dans lequel le phénol lui-même sert de solvant.